# FASCICULE DE BREVET EUROPEEN

(11) **EP 0 925 570 B1**
(45) Date de publication et mention de la délivrance du brevet: **16.07.2003**
(21) Numéro de dépôt: 97901114.5
(22) Date de dépôt: 17.01.1997
(51) Int. Cl.: G09B 21/00

(54) **DISPOSITIF PORTATIF DE LECTURE POUR NON-VOYANT**
TRAGBARE LESEEINRICHTUNG FÜR BLINDE
PORTABLE READING DEVICE FOR THE BLIND

(43) Date de publication de la demande: 30.06.1999
(73) Titulaire: Parienti, Raoul, 06000 Nice (FR)
(72) Inventeur: Parienti, Raoul, 06000 Nice (FR)
(74) Mandataire: Bonneau, Gérard
(86) Numéro de dépôt international: FR9700096
(87) Numéro de publication internationale: WO97026639

(56) Documents cités:
- EP-A- 0 542 054
- DE-A- 3 901 023
- FR-A- 2 493 569
- FR-A- 2 598 316
- US-A- 3 594 787
- US-A- 3 993 407
- IEEE TRANSACTIONS ON INSTRUMENTATION AND MEASUREMENT, vol. 33, no. 2, Juin 1984, NEW YORK, USA, pages 122-126, XP002016997 D. THONG NGUYEN ET AL.: "A Vocalized Color Recognition System for the Blind"

## Description

La présente invention décrit un dispositif portatif de lecture de texte imprimé de façon traditionnelle pour les non-voyants.

Le développement spectaculaire des techniques informatiques et électroniques a permis d'offrir à un public de plus en plus large des appareils de plus en plus performants à des coûts attractifs : ordinateur, scanner, imprimante, fax représentent les outils de la vie courante.

Malheureusement, ces progrès n'ont pas eu de répercussion pour les non-voyants, notamment pour l'accès à la lecture et plus largement à la culture et à la connaissance.

Certes, le non-voyant peut suivre les émissions radio pour avoir accès à l'information culturelle ou autre. Mais, à ce jour, l'accès à une information choisie par ses soins lui est quasiment interdit.

On a vu ça et là quelques expérimentations en vue de permettre à l'aveugle d'accéder au livre par le biais de cassettes audio préenregistrées. Puis, plus récemment, quelques expérimentations ont été conduites pour permettre à l'aveugle l'accès à la lecture par saisie de texte par scanner et traitement des données permettant de faire apparaître ledit texte saisi sur des planches en Braille, le brevet EP-A-0 542 054 décrit ce type de dispositif.

Mais ces procédés sont encombrants et coûteux et ne donnent aux non-voyants aucune réelle autonomie.

La méthode des points saillants inventés par Louis Braille est à ce jour assez diffusée. Les non-voyants entraînés à lire en Braille sont capables d'une vitesse de lecture très élevée et d'un niveau d'intégration remarquable. Malheureusement le nombre de livres retranscrits en Braille est très limité et leur encombrement tout comme leur prix est important.

Quelques tentatives de procédés portatifs ont été expérimentées. Le brevet DE-A-39 01 023 décrit un appareil portatif que le non-voyant fait défiler sur un journal correctement plaqué contre un bord prévu à cet effet, le texte saisi étant transcrit sur une ligne en Braille.

Ce brevet décrit un dispositif qui présente cependant plusieurs inconvénients dans la mesure où l'exploitation par le non-voyant du texte transcrit en Braille ne s'effectue pas exactement en même temps que la saisie :
- nécessite un dispositif de guidage,
- reste encombrant.

L'invention décrite dans le présent brevet permet de remédier à tous ces inconvénients.

En effet, ladite invention permet à l'aveugle de lire n'importe quel ouvrage, journal ou document imprimé sur tout support en caractères traditionnels sans aucun accessoire, grâce à un dispositif miniature qui se présente sous la forme d'un étui introduit à l'extrémité de l'index à l'instar d'un dé à coudre.

L'invention concerne un dispositif portatif de lecture pour aveugles, intégrant des capteurs optiques capables de saisir un texte imprimé, une électronique mémorisant des logiciels adaptés pour reconnaître les caractères imprimés, ainsi que des logiciels de conversion desdits caractères imprimés en caractères Braille, et une zone de reconnaissance tactile, caractérisé en ce que le dispositif de lecture est constitué d'un micro boîtier fixé grâce à un étui à l'extrémité de l'index du non-voyant à l'instar d'un dé à coudre, ledit micro boîtier, comprenant une matrice de capteurs optiques, une surface tactile; une unité électromagnétique conçue pour afficher en Braille, caractère par caractère aussitôt que l'index du non-voyant glisse sur un caractère imprimé; et lorsque ledit index se trouve exactement au-dessus dudit caractère; cette unité électromagnétique intègre six électro-aimants destinés à reproduire les caractères Braille et deux autres destinés à indiquer au lecteur un défaut de guidage, ces derniers sont activés si le non-voyant dévie son index vers le bas ou vers le haut lorsqu'il le déplace le long d'une ligne d'un texte.

L'objet de l'invention sera mieux compris à la lecture de la description détaillée et en référence aux dessins :
- la figure 1 représente une vue d'ensemble de l'invention, avec l'étui, le boîtier solidaire de l'étui est connecté à un boîtier électronique fixé au poignet,
- la figure 2 représente une vue plus détaillée du boîtier de lecture avec une matrice de capteurs optiques, une unité de reconnaissance tactile comprenant des électro-aimants et une optique,
- la figure 3 montre un autre exemple de fixation du boîtier électronique sur la ceinture laquelle comprend la source d'énergie,
- la figure 4 montre un détail de connexion entre le boîtier électronique et la ceinture,
- la figure 5 montre une vue éclatée du boîtier de lecture,
- la figure 6 montre un détail de l'action du noyau de l'électro-aimant oeuvrant avec la tige fixe,
- la figure 7 montre un agrandissement du détail cité dans la figure 6, mettant en évidence l'action des petits points (tiges fixes)et des grands points (noyaux mobiles) dans la lecture en Braille, ainsi que l'action du ressort à lame,
- la figure 8 montre l'action des indicateurs de positionnement mobiles situés en haut et en bas de la zone tactile,
- la figure 9 montre une coupe transversale mettant en évidence les bossages latéraux,
- la figure 10 montre une mise en oeuvre particulière permettant d'actionner les petits points et les gros points de l'écriture Braille, les tiges devenant ainsi mobiles et comportant sur la partie inférieure un noyau ferreux solidaire des tiges,
- la figure 11 montre l'action de nombreux points formant une surface de type « graphique »,
- la figure 12 montre un synoptique de l'électronique du boîtier,
- la figure 13 montre un exemple de partition du champ visuel en matrice, ainsi que les axes médians associés.

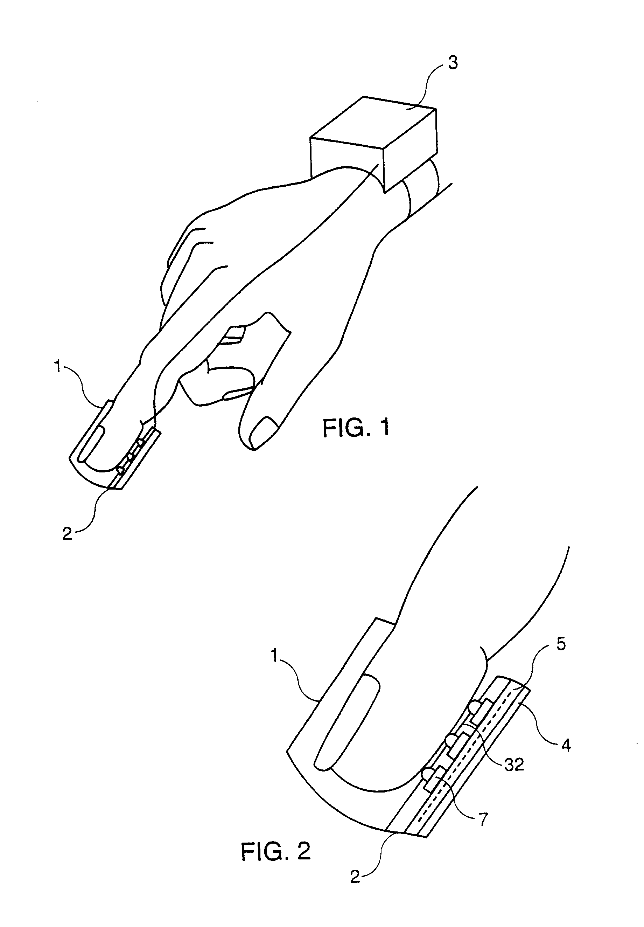

L'invention se compose d'un étui (1) destiné à être placé préférentiellement sur l'extrémité de l'index du non-voyant, mais peut également se décliner en plusieurs étuis placés sur plusieurs doigts simultanément. Le micro boîtier de lecture (2) est solidarisé à l'étui (1). L'ensemble étui (1), micro boîtier (2) est relié à un boîtier électronique (3), figure 1.

Voyons plus en détail les trois sous-ensembles du micro boîtier (2), figure 2, il comprend :
- une lentille transparente (4) destinée à glisser sur le texte,
- une matrice de capteurs optiques (5) constitué par une micro-caméra à base d'une matrice de pixels,
- une partie électro-mécanique (6), constituant l'unité de reconnaissance tactile, qui comprend une surface tactile (32) en contact permanent avec la pulpe de l'index du non-voyant, ladite unité de reconnaissance tactile est pourvue dans sa version de base de huit micro électro-aimants (7), six sont destinés à reproduire les caractères Braille et deux sont destinés à indiquer au lecteur un défaut de guidage. Ces deux électro-aimants de guidage (27) et (28) seront activés si le non-voyant dévie son index vers le bas ou vers le haut lorsqu'il le déplace le long d'une ligne d'un texte.

Pour détecter une éventuelle déviation du doigt du non-voyant au cours de la lecture, le guidage de référence est réalisé grâce aux lignes blanches de chaque interligne. A cet effet, l'électronique (3) intègre un logiciel de positionnement qui, en plus de la reconnaissance des caractères et à leur transcription en Braille, permet d'identifier les interlignes dans un paragraphe, ou toute zone blanche encadrant un texte, afin de stimuler en cas de déviation, les électro-aimants (27) ou (28), selon un code pré-défini.

De plus, le logiciel de positionnement est capable de guider le non-voyant lorsqu'il devra passer à la ligne suivante à la fm de la lecture de chaque ligne. Alors que seuls les caractères saisis par la partie centrale des capteurs (5) sont transcrits en Braille, les caractères des lignes supérieures et inférieures sont saisis en permanence, afin d'identifier la fin de chaque ligne et le début exact de la ligne suivante. Un codage particulier des stimulations tactiles indique au non-voyant la fin d'une ligne : par exemple, l'action simultanée des électro-aimants, (27) et (28). Le guidage, permettant au non voyant d'attaquer exactement le début d'une ligne, s'effectue comme précédemment par la stimulation haute ou basse des mêmes électro-aimants (27) et (28).

Grâce à la présente invention, tout se passe pour l'aveugle, comme si tous les caractères étaient directement imprimés en Braille.

Rappelons que l'écriture Braille est constituée essentiellement de 80 caractères comprenant l'ensemble de l'alphabet avec indication des majuscules, ponctuation, chiffres et opérateurs de calcul, ainsi que d'autres caractères moins courants.

Chaque caractère est constitué par la combinaison de gros points et de petits points, grâce à une matrice composée de 3 lignes et de 2 colonnes. En fait, seuls les gros points en relief représentent les caractères, les petits points en relief également, ne servent qu'à indiquer la position des gros points dans chaque groupe de 6. A ce jour les moyens informatiques modernes n'utilisent dans les planches à Braille que les gros points. L'invention décrite dans le présent document utilise indifféremment les deux méthodes.

Le principe de fonctionnement est le suivant : pour lire un texte, l'aveugle n'a qu'à faire glisser son index pourvu du micro boîtier (2) le long du texte, aussitôt lesdits caractères sont saisis par la micro-caméra, une électronique (3) adaptée va reconnaître les caractères alphanumériques, et va, aussitôt transmettre les signaux utiles aux micro électro-aimants (7) de la partie mécanique (6).

Chaque caractère imprimé, saisi par les capteurs optiques (5) et reconnu par l'électronique (3), provoque la mise en relief des gros points sous l'index de l'aveugle. Ainsi, chaque caractère imprimé sur un texte va apparaître en Braille, grâce à l'action des micro électro-aimants pilotés par une unité de traitement électronique intégrée dans le boîtier (3).

Décrivons maintenant un mode de réalisation particulier et plus détaillé de l'invention : celle-ci est constituée d'un étui (1) destiné à envelopper l'extrémité de l'index de l'utilisateur. Fixé audit étui un boîtier de lecture (2) comprend les trois sous ensembles définis plus haut (4), (5), et (6).

L'ensemble de capteurs optiques (5) est protégé par une lentille transparente (4), réalisée en verre ou en matière non minérale, faisant office de protection des capteurs (5), ainsi que d'optique de mise au point.

Lorsque l'optique (4) glisse sur les caractères d'un texte, ceux-ci sont saisis par les pixels des capteurs optiques (5), qui en exploitent les signaux, soit par nuance de gris (monochrome), soit selon les différentes couleurs.

Les signaux recueillis par la matrice de capteurs optiques (5), sont acheminés vers le boîtier électronique (3). Ce boîtier peut être porté à la taille comme un baladeur ou au poignet comme une grosse montre, figure 1, ou encore dans une quelconque poche de l'utilisateur.

Notons cependant que la ceinture proche du centre de gravité d'un individu, présente de ce fait l'endroit du corps le plus adapté pour porter une charge avec le minimum de fatigue.

Selon une version de l'invention la ceinture comprend l'alimentation du boîtier électronique (3) par accumulateurs rechargeables (9) classiques, ou mieux souples de type lithium ou autre, ainsi que le circuit spécifique (10) permettant le rechargement desdits accumulateurs, par simple introduction des tiges de bouclage (12) dans une prise de courant du secteur. Cette commodité simplifiera grandement la vie des non-voyants.

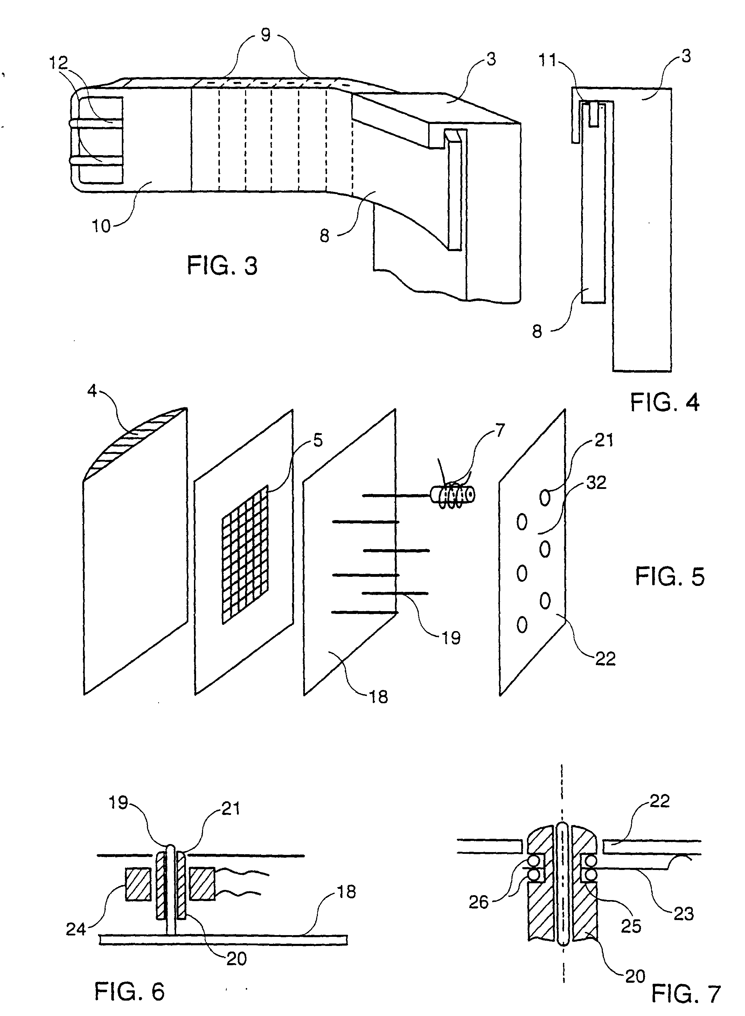

Ainsi le boîtier (3) qui intègre l'électronique de l'invention va se fixer préférentiellement sur la ceinture (8), et se connecter à l'alimentation, grâce à un couple de prises mâles et femelles adapté (11) figure 3 et 4. Bien entendu la ceinture (8) pourvue de son alimentation et de son système de rechargement peut alimenter d'autres boîtiers tels que baladeur ou autre.

Décrivons maintenant les différents sous-ensembles de l'électronique du boîtier (3). Celui-ci intègre un microprocesseur (13) cadencé par une horloge (14), ledit microprocesseur gère une mémoire vive RAM (15), et une mémoire morte ROM (16). La mémoire ROM va intégrer un logiciel capable de reconnaître les caractères, de type « OCR », et un logiciel capable de faire correspondre à tout caractère son homologue en Braille. A la reconnaissance de chaque caractère saisi par les capteurs optiques (5), le microprocesseur (13) donne les instructions nécessaires à la délivrance des impulsions électriques, via un circuit logique (17), vers les micro électro-aimants (7), afin de faire apparaître en Braille sous l'index de l'utilisateur qui se trouve dans l'étui (1) le caractère saisi par les capteurs optiques(5).

Selon une version plus sophistiquée de l'invention, l'optique (4) peut également saisir des caractères à distance, sans contact avec le texte à « traduire » en Braille. Pour ce faire, l'optique (4) peut soit être interchangeable, soit intégrer un système « autofocus » utilisant des procédés bien connus, tels que l'analyse du contraste ou l'émission infrarouge, afin de déterminer la distance optique/texte, pour effectuer la mise au point ad hoc.

La mise au point peut être réalisée grâce à une focale fixe en utilisant les propriétés de l'hyperfocale, permettant d'obtenir une profondeur de champ suffisante, afin d'avoir une image nette, du contact de la lentille sur le texte jusqu'à plusieurs centimètres de distance.

La capacité de l'optique de lire des caractères à distance permettra, notamment de pouvoir traiter des caractères de grandes dimensions, tel que titres de livres.

Nous allons décrire maintenant un des éléments principaux de l'invention, à savoir le module mécanique conçu pour communiquer les signaux tactiles aux non-voyants.

Jusqu'à ce jour, le non-voyant devait déplacer son doigt le long d'un texte transcrit en Braille. Les points saillants caractérisant le Braille, sont généralement réalisés par repoussage dans un papier spécial, ou selon d'autres procédés plus récents, sur des tablettes à picots. Cependant pour lire le Braille, le doigt du non-voyant devait invariablement se déplacer sur une surface comportant l'ensemble des points en relief.

L'invention présentée permet de réduire la surface tactile communiquante à sa plus simple expression, à savoir la surface de l'extrémité de l'index .

Le non-voyant perçoit ainsi les signaux tactiles grâce à l'action des électro-aimants.

Pour parfaire le réalisme de la sensation tactile liée au déplacement virtuel du doigt, les électro-aimants ne seront pas actionnés simultanément pour représenter chaque lettre, mais avec un léger décalage :

L'action des points saillants de la première colonne étant activée quelques fractions de secondes, avant ceux de la deuxième colonne, et inversement ceux de la deuxième disparaissant quelques fractions de secondes après ceux de la première colonne, pour faire apparaître la lettre suivante.

Nous allons décrire en détail quelques modes de réalisation non exhaustifs donnés à titre d'exemple.

Selon le mode de réalisation le plus simple de l'invention, la surface tactile de celle-ci est pourvue de 6 points mobiles, figure 2.

Un autre mode de réalisation met en oeuvre 6 petits points fixes et 6 gros points mobiles.

La figure 5 nous montre une vue éclatée du boîtier (2), réalisé selon ce dernier mode et notamment la plaquette (18) sur laquelle sont solidarisées 6 tiges non métalliques (19). L'extrémité de chaque tige est destinée à être saillante en permanence faisant office de petits points de repérage. Les gros points représentant les lettres, sont en fait les noyaux (20) mobiles des micro électro-aimants (7), l'extrémité de ces noyaux étant arrondie. Chaque noyau (20), est creux et traversé par la tige non métallique (19), ainsi au repos, seuls les points de repérage sont saillants et sortent des six trous (21), se trouvant sur la plaque supérieure (22) de la surface tactile (32), voir figure 6. Quand un micro électro-aimant est en action, le gros point constitué du noyau mobile (20) va devenir saillant et de ce fait le petit point va disparaître, figure 7. Un ressort à lame (23) va rappeler le noyau de l'électro-aimant à sa position de repos quand cesse l'action du courant sur la bobine (24) de l'électro-aimant. Le ressort à lame peut avantageusement agir sur une gorge (25) du noyau (20), en pressant sur 2 rondelles toriques souples (26), figure 7.

L'inertie résultant de la déformation des rondelles souples permettra aux mouvements rapides du noyau (20), d'avoir une certaine progressivité. Cette inertie combinée avec le léger décalage entre la mise en action des points de la première colonne et de la deuxième colonne décrit précédemment, donnera au non-voyant une perception très réaliste du déplacement virtuel du doigt sur les caractères en relief.

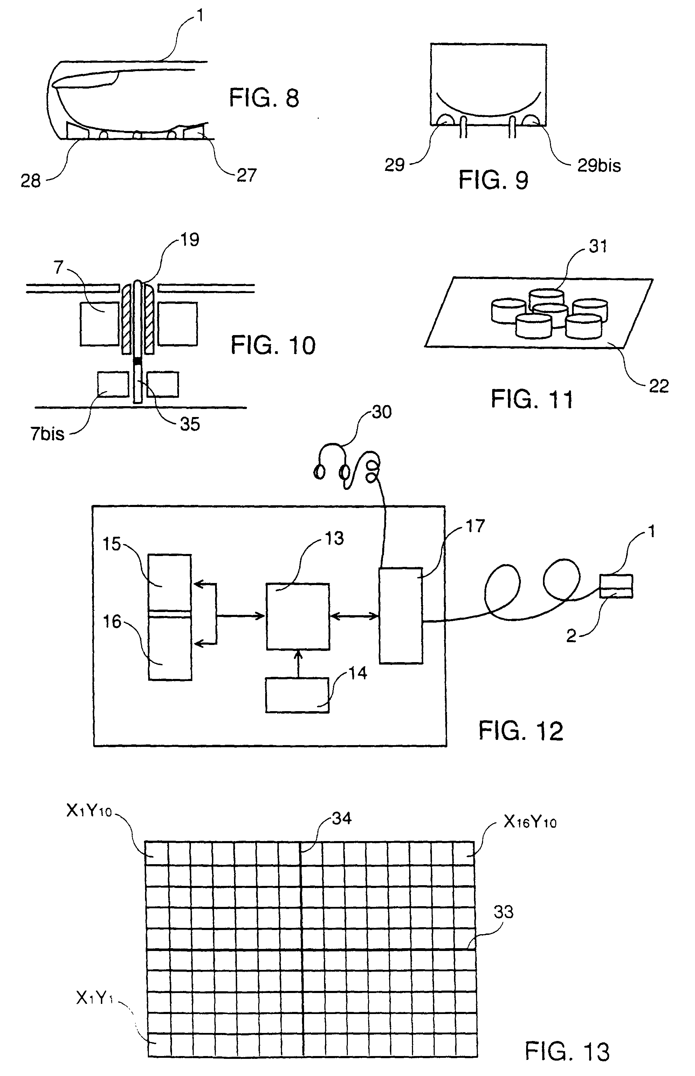

La figure 10 montre une disposition particulière des deux électro-aimants (7) et (7 bis), respectivement pour le gros point et pour le petit point. Dans ce mode de réalisations la surface tactile (32) est dépourvue de tout relief au repos, les points petits ou gros apparaissant à chaque lettre en Braille.

Selon un autre mode de réalisation particulière de l'invention, une multitude de tiges mobiles (31) seront mises en oeuvre. Chaque petit point est représenté par la mise en relief d'une tige, chaque gros point est représenté par la mise en relief de plusieurs tiges, figure 11. Ce mode de réalisation plus complexe de l'invention permettra de procurer au non-voyant un confort et un réalisme optimum du toucher.

En effet, dans ce mode de réalisation les petits points et les gros points des caractères Braille ne vont pas apparaître subitement sous le doigt du non-voyant, mais vont évoluer progressivement de droite à gauche de l'index, avec un effet de vague, reproduisant exactement la sensation de lecture par déplacement du doigt sur les caractères en relief.

De plus ce mode de réalisation permettra également de « lire » les dessins au fur et à mesure que le doigt du non-voyant passe dessus. Les capteurs optiques (5) vont saisir les contours du dessin qui vont se reproduire en relief sous le doigt du non-voyant en temps réel grâce à l'action des électro-aimants (7) sur les tiges.

Dans une version plus élaborée de ce mode de réalisation, les capteurs optiques (5) sont capables d'apprécier différents niveaux de contrastes des contours du dessin à saisir, de sorte que l'électronique du boîtier (3) transmettra corrélativement différents niveaux d'intensité de courant agissant sur les électro-aimants (7), afin d'obtenir plusieurs niveaux de relief des tiges (31), en fonction de l'image à transcrire.

Comme il a été dit plus haut, l'invention est pourvue d'un dispositif de guidage : pour suivre une ligne du texte convenablement, la partie supérieure et inférieure de la surface tactile (32) sera pourvue d'un indicateur de positionnement mobile (27) et (28). Si au cours de la lecture, le déplacement du doigt du non-voyant s'écartait légèrement vers le bas d'une ligne, le système optique détectera ce décalage et sur ordre du microprocesseur (13) le positionneur inférieur (27) va se soulever toujours grâce à l'action d'un électro-aimant, figure 8. Cette action invitera le lecteur à corriger sa trajectoire légèrement vers le haut. L'indicateur de positionnement supérieur (28) fonctionnera sur le même principe, si l'utilisateur s'écarte de la ligne vers le haut.

Pour détecter le décalage vers le haut ou vers le bas, la matrice sera pourvue d'un ensemble de capteurs optiques centraux et d'un ensemble de capteurs optiques périphériques qui permettront d'identifier les interlignes et les zones blanches encadrant le texte.

L'image des caractères doit impressionner les capteurs exclusivement sur la partie centrale, le décalage de l'image des caractères sur les capteurs périphériques hauts ou bas, va provoquer la simulation des capteurs de positionnement respectivement haut et bas.

Si la surface tactile est pourvue en haut et en bas d'indicateurs de positionnement, la partie droite et gauche sera quant à elle pourvue d'un bossage statique (29) et (29 bis). Chacun des bossages est pourvu d'un capteur de pression, une légère pression sur le capteur de droite (29) va provoquer le déplacement virtuel des caractères en Braille sous l'index, une pression plus prononcée va accélérer le défilement desdits caractères, bien entendu, si ces derniers ont déjà été saisis par le capteur et mémorisés dans la mémoire tampon de la RAM (15), en revanche une légère pression sur le capteur de gauche (29 bis) va stopper le défilement et une pression plus accentuée sur le même capteur, va faire défiler les caractères à l'envers simulant ainsi un déplacement du doigt, non pas de gauche à droite, mais de droite à gauche et inversement pour les langues se lisant de droite à gauche. L'ensemble des fonctions qui viennent d'être décrites résulte de l'interprétation des signaux des capteurs (29) et (29 bis) par le microprocesseur (13).

Selon une variante complémentaire de l'invention qui vient d'être décrite, un signal audible va compléter l'information en Braille que va exploiter le non-voyant. Ainsi parallèlement au déchiffrement du texte, grâce à la saisie des caractères et à leur traduction en Braille, un écouteur discret (30) connecté à l'électronique, va émettre une série de signaux acoustiques à l'oreille du non-voyant. L'émission de ces signaux sonores permettra au non-voyant, dans un premier temps, de prendre connaissance de la mise en page du texte. En effet, le texte à lire peut être entrecoupé d'images et être disposé en une ou plusieurs colonnes. Avant la lecture, l'utilisateur va parcourir rapidement avec son index pourvu du dispositif, les différents champs de la page « à lire », les passages blancs ou colorés vont être traduits par l'électronique par une série de tonalités spécifiques, que l'utilisateur aura à apprendre dans un premier temps. Après avoir mémorisé la mise en page du texte, le non-voyant pourra effectuer la saisie des caractères avec efficacité. Par ailleurs un signal sonore permettra d'apprécier la taille des caractères, par exemple plus grave pour les grandes lettres, plus aiguë pour les petites lettres, afin de permettre au non-voyant de pondérer la vitesse de défilement de son doigt sur le texte.

Selon une version plus élaborée de l'invention la matrice de capteurs optiques est conçue pour reconnaître les couleurs.
La correspondance couleur visible / fréquence audible, évoquée plus haut n'est pas qu'une simple vue de l'esprit, le terme « chromatique » s'utilise indifféremment pour l'étude des couleurs ou en musique. Le système chromatique en musique est basé sur la division d'une octave en 12 parties égales.
Des études ont été menées afin de mettre en évidence la correspondance pouvant exister entre les couleurs et les sons.

A titre indicatif, nous donnerons ci-après un exemple de correspondance entre les couleurs et les fréquences sonores proposé dans différents traités :
si le Do central d'un piano est accordé sur une fréquence de 256 périodes par seconde (Hertz), la note Sol immédiatement à gauche du Do central sera accordée à 192 Hertz et correspond au rouge foncé, et en allant vers les notes plus aiguës :
- la note Sol # correspond au rouge,
- la note La correspond au rouge-orange,
- la note La # correspond à l'orange,
- la note Si correspond au jaune,
- la note Do correspond au jaune-vert,
- la note Do # correspond au vert,
- la note Ré correspond au vert-bleu,
- la note Ré # correspond au bleu,
- la note Mi correspond au bleu-violet,
- la note Fa correspond au violet,
et à nouveau le Sol de l'octave suivante correspond au rouge foncé et ainsi de suite.

Lorsque le doigt du non-voyant va glisser sur les caractères, la stimulation en Braille sur son doigt s'effectuera de façon concomitante à l'émission par l'écouteur (30) d'un signal sonore pré-défini correspondant à la couleur dominante.

Lorsque le non-voyant va effectuer une lecture sans contact du titre d'un livre par exemple, le signal sonore va lui indiquer que la couleur dominante de la couverture est rouge et que le titre est imprimé en blanc.

Une version plus élaborée de l'invention consiste à effectuer un balayage de la matrice des capteurs optiques en affectant à chaque module associé un ou plusieurs pixels, la fréquence sonore pré-définie correspondante, la puissance du signal sonore étant pondérée par l'intensité lumineuse du module balayé.

Sur une matrice élémentaire de 10 x 16, par exemple, allant de (x₁y₁) à (x₁₆y₁₀), l'électronique analyse, par exemple, d'abord le module (x₁y₁) puis le long de la ligne horizontale (x₁y₁) jusqu'à (x₁₆y₁) et ainsi de suite jusqu'à la dernière ligne de (x₁y₁₀) à (x₁₆y₁₀), (figure 13).

Le saut d'une ligne à une autre et d'une image à une autre sera caractérisé par un signal sonore spécifique, ou un silence très court, quelques millisecondes pour le passage d'une ligne à une autre, et quelques dizaines de millisecondes pour le passage d'une image à une autre.

Dans ce procédé de balayage du « champ visuel » l'ensemble du champ sera ainsi analysé et chaque fois que dans ledit champ des signes alphanumériques seront saisis, l'électronique du système va les reconnaître et va transmettre les impulsions correspondantes vers les électro-aimants (7) stimulant ainsi l'index de l'utilisateur par la traduction des caractères saisis en Braille.

Selon une version encore plus élaborée de l'invention, celle-ci est pourvue d'une optique capable d'effectuer une mise au point sur des distances plus importantes que quelques centimètres, ainsi l'utilisateur pourra par intégration des sons musicaux associés à chaque module du champ, et par la prise de connaissance des caractères alphanumériques compris dans le champ, grâce à la stimulation en Braille de l'index du non-voyant, ce dernier disposera d'une nouvelle autonomie. Il pourra par exemple prendre connaissance que la vitrine qui est devant lui est de couleur dominante jaune et que ladite vitrine est surmontée d'une inscription boulangerie à dominante rouge.

Dans une version particulière de l'invention, les capteurs optiques (5) permettent de détecter la « lumière » infrarouge et la reproduisent par une fréquence sonore spécifique, indiquant au non-voyant la présence d'êtres humains ou d'animaux ou de source de chaleur.

Pour donner une acuité supérieure de l'image, l'analyse de cette dernière va en privilégier le centre. Aussi, l'image est séparée par deux médianes et lors de l'analyse module par module, la puissance du signal acoustique en corrélation avec la couleur dominante associée à chaque module (xₙyₘ), déjà pondérée par la luminosité du module, est uniformément croissante en se rapprochant des axes médians, tant de l'axe vertical (34), que de l'axe horizontal (33) et s'atténue uniformément en s'en éloignant.

L'accroissement de la puissance du signal sonore peut être de l'ordre de 1,5 décibels sur l'axe médian vertical et de 3 décibels sur l'axe médian horizontal. Le gain de puissance au centre de l'image procure un confort supplémentaire et proche de la vision naturelle. En effet, la vision naturelle privilégie la vue de ce qui est devant au détriment du champ qui s'en écarte.

Par ailleurs, l'accroissement de la puissance vers le centre de l'image permet une analyse plus rapide des images relativement pauvres en informations. Si, par exemple, dans l'analyse de l'image vue plus haut, les modules (x₁y₁), (x₁y₂) jusqu'à (x₁y₇) reçoivent un signal lumineux identique, la partie de l'image analysée corrélative étant invariante, l'électronique ne va pas, après analyse de chacun des sept modules de la colonne, émettre consécutivement sept signaux sonores identiques, mais un signal unique dont la variation de puissance permettra au non-voyant de repérer la zone balayée.

Cette particularité permettra au non-voyant d'appréhender très rapidement un champ pauvre en informations visuelles et inversement de s'attarder lorsque le champ est plus riche en informations. Dans cette version, le dispositif de capteur optique associé à la surface tactile peut avantageusement en être différencié. L'ensemble des capteurs optiques de cette dernière version peut être fixé sur une simple paire de lunettes, par ailleurs dans cette version, il y aura deux matrices de capteurs optiques, une pouvant se situer au niveau de l'oeil droit et l'autre au niveau de l'oeil gauche. Dans cette version binoculaire toujours associée à la lecture en Braille par l'index du non-voyant des caractères alphanumériques apparaissant sur la matrice de capteurs optiques (5), la perception simultanée des sons résultants de l'analyse des signaux des capteurs matriciels placés sur l'oeil droit vers l'oreille droite, et des sons résultants de l'analyse des signaux des capteurs placés sur l'oeil gauche vers l'oreille gauche, procure au non-voyant une perception stéréophonique, à partir de quoi, avec un peu d'entraînement, il pourra appréhender une notion de l'espace et à terme une perception quasi stéréoscopique.

La perception quasi stéréoscopique ainsi proposée, combinée à la lecture en Braille de l'ensemble des caractères alphanumériques sont de nature à procurer aux non-voyants un nouvel espace d'autonomie et de liberté.

Enfin, pour obtenir une version déclinée de l'invention et en utilisant des techniques connues déjà mises en oeuvre, il est possible d'intégrer dans la mémoire ROM (16) un logiciel de synthèse de la parole associé à un dictionnaire de mots et à un dictionnaire de phonèmes correspondants, de sorte que concomitamment à la lecture en Braille le non-voyant peut écouter le texte saisi par les capteurs optiques (5), qui peut par ailleurs être traduit dans une langue de son choix grâce à un logiciel de traduction adéquat.

Les formes, dimensions et dispositions des différents éléments, ainsi que les matières utilisées pour la fabrication, pourront varier dans la limite des équivalents, sans changer pour cela la conception générale de l'invention, qui vient d'être décrite.

## Revendications

1. Dispositif portatif de lecture pour non-voyants, intégrant des capteurs optiques capables de saisir un texte imprimé, une électronique mémorisant des logiciels adaptés pour reconnaître les caractères imprimés, ainsi que des logiciels de conversion desdits caractères imprimés en caractères Braille, et une zone de reconnaissance tactile, **caractérisé en ce que** le dispositif de lecture est constitué d'un micro boîtier (2) fixé à un étui introduit à l'extrémité de l'index du non-voyant à l'instar d'un dé à coudre, ledit micro boîtier (2), comprenant une matrice de capteurs optiques (5), pourvue d'une lentille (4), et une unité électromagnétique (6), conçue pour afficher en Braille, caractère par caractère, sur une surface tactile (32), aussitôt que l'index du non-voyant glisse sur un caractère imprimé et lorsque ledit index se trouve exactement au-dessus dudit caractère; ladite unité électromagnétique (6) intègre six électro-aimants (7) destinés à reproduire les caractères Braille et deux autres (27) et (28), destinés à indiquer au lecteur un défaut de guidage; ces derniers étant activés si le non-voyant dévie son index vers le bas ou vers le haut lorsqu'il le déplace le long d'une ligne d'un texte.

2. Dispositif portatif de lecture pour non-voyant selon la revendication 1 **caractérisé en ce que** un boîtier électronique (3) intègre un logiciel permettant d'identifier les interlignes dans un paragraphe, ou toute zone blanche encadrant un texte, afin de stimuler les électro-aimants (27) ou (28), selon un code pré-défini en cas de déviation de l'index au cours de la lecture.

3. Dispositif portatif de lecture pour non-voyant selon la revendication 1 ou 2 **caractérisé en ce que** les caractères des lignes supérieures et inférieures du texte imprimé sont saisis en permanence par les capteurs (5), mémorisant ainsi le début et la fin de chaque ligne, de sorte que des stimulations codées des électro-aimants (27) et (28) indiquent au non-voyant la fin de ligne et le positionnement très exacte de la ligne suivante.

4. Dispositif portatif de lecture pour non-voyant selon la revendication 1 **caractérisé en ce que** la mise en action des gros points constitués par des noyaux mobiles (20), représentant les lettres, fait disparaître les petits points fixes, constitués par des tiges (19).

5. Dispositif portatif de lecture pour non-voyant selon la revendication 1 et 3, **caractérisé en ce que** chaque noyau mobile (20) est creux et traversé par la tige fixe (19).

6. Dispositif portatif de lecture pour non-voyant selon l'une quelconque des revendications précédentes, **caractérisé en ce qu'**il est mis en oeuvre une multitude de tiges mobiles (31); chaque petit point étant représenté par la mise en relief d'une tige, chaque gros point par la mise en relief de plusieurs tiges.

7. Dispositif portatif de lecture pour non-voyant selon l'une quelconque des revendications précédentes, **caractérisé en ce que**, lorsque le non-voyant glisse son doigt sur une image, les capteurs optiques (5) sont capables d'apprécier différents niveaux de contrastes des contours de ladite image, de sorte que l'électronique du boîtier (3) transmet en temps réel les différents niveaux d'intensité de courant agissant sur les électro-aimants (7), afin d'obtenir plusieurs niveaux de relief des tiges (31) pour représenter une image.

8. Dispositif portatif de lecture pour non-voyant selon l'une quelconque des revendications précédentes, **caractérisé en ce que** la partie droite et gauche de la surface tactile (32) est pourvue de bossage (29) et (29 bis) dont la pression provoque en fonction de la direction et de l'intensité, l'accélération du défilement virtuel des caractères, l'arrêt du défilement ou le défilement à l'envers.

9. Dispositif portatif de lecture pour non-voyants selon l'une quelconque des revendications précédentes, **caractérisé en ce que** l'optique (4) est capable de lire les caractères à distance en utilisant un système autofocus ou l'hyperfocal d'un objectif fixe.

10. Dispositif portatif de lecture pour non-voyant selon l'une quelconque des revendications précédentes, **caractérisé en ce qu'**un balayage de chaque module du champs par la matrice de capteurs optiques (5) affecte audit module associé à un ou plusieurs pixels, une fréquence sonore pré-définie correspondant à la couleur dominante dudit module.

11. Dispositif portatif de lecture pour non-voyant selon l'une quelconque des revendications précédentes, **caractérisé en ce que** le saut d'une ligne à une autre et d'une image à une autre, lors du balayage par les capteurs (5), est marqué par un signal sonore spécifique ou un silence très court, quelques millisecondes pour le passage d'une ligne à une autre, et quelques dizaines de millisecondes pour le passage d'une image à une autre.

12. Dispositif portatif de lecture pour non-voyant selon l'une quelconque des revendications précédentes, **caractérisé en ce que** les capteurs optiques (5) permettent de détecter la lumière infrarouge et la reproduisent par une fréquence sonore spécifique, indiquant au non-voyant la présence d'êtres humains ou d'animaux.

13. Dispositif portatif de lecture pour non-voyant selon l'une quelconque des revendications précédentes, **caractérisé en ce que** la puissance du signal acoustique en corrélation avec la couleur dominante associée à chaque module (XₙYₘ) est uniformément croissante en se rapprochant des axes médians (33) et (34), et s'atténue uniformément en s'en éloignant.

14. Dispositif portatif de lecture pour non-voyant selon l'une quelconque des revendications précédentes, **caractérisé en ce qu'**une matrice de capteurs optiques se situe au niveau de chaque oeil, les sons résultant de l'analyse des signaux par la matrice des capteurs optiques (5) placés sur l'oeil droit sont dirigés vers l'oreille droite et les sons résultant de l'analyse des signaux par la matrice des capteurs optiques (5) placés sur l'oeil gauche sont dirigés vers l'oreille gauche, procurant au non-voyant une perception stéréophonique.

15. Dispositif portatif de lecture pour non-voyant selon l'une quelconque des revendications précédentes, **caractérisé en ce que** une ceinture comprend l'alimentation du boîtier électronique (3) par accumulateurs rechargeables (9), ainsi qu'un circuit spécifique (10), permettant le rechargement desdits accumulateurs par simple introduction de tiges de bouclage (12) dans une prise de courant du secteur.

## Claims

1. A portable reading device for the blind including optical sensors capable of acquiring printed text, electronics with built-in software for printed character recognition and software for converting said printed characters into Braille characters, and a touch recognition surface, **characterized in that** the reading device comprises a miniature housing (2) attached to a sleeve to be fitted over the tip of the blind person's index finger like a thimble, said miniature housing (2) including an optical sensor array (5) provided with a lens (4), and an electromagnet unit (6) for displaying in Braille one character at a time, on a touch surface (32), as soon as the blind person's finger slides over a printed character and when said index finger is exactly located over said character; said electromagnetic unit (6) contains six electromagnets (7) for reproducing Braille characters and two further electromagnets (27) and (28) for indicating incorrect tracking to the reader; the latter two electromagnets being activated when the blind person moves his (her) finger too far up or down as it runs along a line of text.

2. A portable reading device for the blind according to claim 1 **characterized in that** an electronic box (3) has built-in software for identifying line spacing within a paragraph, or any blank area surrounding text, for stimulating the electromagnets (27) or (28) according to a predetermined code if the finger deviates when reading.

3. A portable reading device for the blind according to claims 1 or 2 **characterized in that** the characters from the lines located just above and below the line being processed are permanently acquired by the sensors (5), thus memorizing the beginning and the end of each line, in such a way that coded stimulation from electromagnets (27) and (28) indicates the end of the line and the accurate position of the next line to the blind person.

4. A portable reading device for the blind according to claim 1, **characterized in that** the activation of large dots representing the letters and made up of mobile cores (20) removes the small fixed dots made up of the rods (19).

5. A portable reading device for the blind according to claims 1 and 3, **characterized in that** each mobile core (20) is hollow and is crossed by the fixed rod (19).

6. A portable reading device for the blind according to any of previous claims, **characterized in that** a plurality of mobile rods (31) is implemented: each small dot being represented by the raising of one rod and each large dot being represented by the raising of several rods.

7. A portable reading device for the blind according to any of previous claims, **characterized in that**, when the blind person moves his (her) finger over an image, the optical sensors (5) can handle several contrast levels of the drawing outlines, in such a way that the electronics of the box (3) sends in real time the various levels of current intensity to the electromagnets (7) to get several levels of raised pattern formed by the rods (31) to transcribe a picture.

8. A portable reading device for the blind according to any of previous claims, **characterized in that** the right and left parts of the touch surface (32) are provided with bosses (29) and (29bis) on which a pressure causes, depending on its direction and intensity, the virtual scrolling of characters to be speed up, stopped or reversed.

9. A portable reading device for the blind according to any of previous claims, **characterized in that** the optical component (4) can read characters remotely by using an autofocus system or the hyper-focal feature of a fixed lens.

10. A portable reading device for the blind according to any of previous claims, **characterized in that** a scanning of each field module by the optical sensor array (5) assigns to said module associated with one or several pixels, a predefined sound frequency corresponding to the dominant colour of said module.

11. A portable reading device for the blind according to any of previous claims, **characterized in that** skipping from a line to a new line or from an image to a new one during the scanning performed by the sensors (5) produces a specific sound signal or a very short silence, the duration of which is a few milliseconds for skipping to the next line and a few tens milliseconds for skipping to the next image.

12. A portable reading device for the blind according to any of previous claims, **characterized in that** the optical sensors (5) can detect infrared light and reproduce it though a specific sound frequency to notify the blind of the presence of human beings or animals.

13. A portable reading device for the blind according to any of previous claims, **characterized in that** the power of the acoustic signal matching the dominant colour associated with each cell (xnym), increases uniformly from the edges to both median axis (33) and (34) and decreases in the reverse direction.

14. A portable reading device for the blind according to any of previous claims, **characterized in that** an optical sensor array is located close to each eye, the sounds resulting from the signals analysed by the optical sensor array (5) placed over the right and the left eye being routed respectively to the right and the left ear, thus providing the blind with stereophonic perception.

15. A portable reading device for the blind according to any of previous claims, **characterized in that** a belt includes the rechargeable battery power supply for the electronic box (3) as well as a specific circuit (10) for charging said batteries by simply plugging the belt buckle rods into a mains socket.

## Patentansprüche

1. Tragbare Lesevorrichtung für Blinde, in die optische Fühler integriert sind, die einen gedruckten Text erfassen können, sowie eine Elektronik, in der Softwares, die dafür ausgelegt sind, die gedruckten Schriftzeichen zu erkennen, sowie Softwares zum Umwandeln dieser gedruckten Schriftzeichen in Braille-Schriftzeichen gespeichert sind, und eine Zone zur Tasterkennung, **dadurch gekennzeichnet, daß** die Lesevorrichtung aus einer Mikroeinheit (2) besteht, die an einer Hülse befestigt ist, die nach der Art eines Fingerhuts auf das Ende des Zeigefingers des Blinden aufgesteckt wird, wobei die Mikroeinheit (2) eine Matrix von optischen Fühlern (5) aufweist, die mit einer Linse (4) versehen ist, sowie eine elektromagnetische Einheit (6), die dafür ausgelegt ist, auf einer Tastfläche (32) Schriftzeichen für Schrift zeichen in Braille-Schrift anzuzeigen, sobald der Zeigefinger des Blinden über ein gedrucktes Schriftzeichen gleitet und wenn der Zeigefinger sich genau über diesem Schriftzeichen befindet, wobei in die elektromagnetische Einheit (6) sechs Elektromagnete (7) integriert sind, die dazu bestimmt sind, die Braille-Schriftzeichen wiederzugeben, sowie zwei andere (27 und 28), die dazu bestimmt sind, dem Leser einen Führungsfehler anzuzeigen, wobei diese zuletzt genannten Elektromagnete aktiviert werden, wenn der Blinde mit seinem Zeigefinger nach unten oder nach oben abweicht, wenn er ihn längs einer Zeile eines Textes bewegt.

2. Tragbare Lesevorrichtung für Blinde nach Anspruch 1, **dadurch gekennzeichnet, daß** in eine Elektronikeinheit (3) eine Software integriert ist, die die Indentifizierung der Durchschüsse in einem Absatz oder jeder einen Text umgebenden Leerzone gestattet, um die Elektromagnete (27 oder 28) nach einem zuvor festgelegten Code im Fall des Abweichens des Zeigefingers während des Lesens anzuregen.

3. Tragbare Lesevorrichtung für Blinde nach Anspruch 1 oder 2, **dadurch gekennzeichnet, daß** die Schriftzeichen der oberen und unteren Zeilen des gedruckten Textes ständig durch die Fühler (5) erfaßt werden, wodurch der Anfang und das Ende jeder Zeile gespeichert wird, so daß codierte Anregungen der Elektromagnete (27 und 28) dem Blinden das Zeilenende und die sehr genaue Positionierung der folgenden Zeile angeben.

4. Tragbare Lesevorrichtung für Blinde nach Anspruch 1, **dadurch gekennzeichnet, daß** die Betätigung der aus beweglichen Kernen (20) bestehenden großen Punkte, die die Schriftzeichen darstellen, die von Stiften (19) gebildeten feststehenden kleinen Punkte verschwinden läßt.

5. Tragbare Lesevorrichtung für Blinde nach Anspruch 1 und 3, **dadurch gekennzeichnet, daß** jeder bewegliche Kern (20) hohl ist und von dem feststehenden Stift (19) durchsetzt wird.

6. Tragbare Lesevorrichtung für Blinde nach einem der vorhergehenden Ansprüche, **dadurch gekennzeichnet, daß** eine Vielzahl von beweglichen Stiften (31) verwendet wird, wobei jeder kleine Punkt durch das Hervorstehen eines Stiftes und jeder große Punkt durch das Hervorstehen von mehreren Stiften dargestellt wird.

7. Tragbare Lesevorrichtung für Blinde nach einem der vorhergehenden Ansprüche, **dadurch gekennzeichnet, daß**, wenn der Finger des Blinden über ein Bild gleitet, die optischen Fühler (5) in der Lage sind, verschiedene Kontraststufen der Umrisse des Bildes einzuschätzen, so daß die Elektronik der Einheit (3) in Echtzeit die verschiedenen auf die Elektromagnete (7) einwirkenden Strompegel überträgt, um zur Darstellung eines Bildes mehrere Reliefhöhen der Stifte (31) zu erhalten.

8. Tragbare Lesevorrichtung nach einem der vorhergehenden Ansprüche, **dadurch gekennzeichnet, daß** der rechte und der linke Teil der Tastfläche (32) mit Erhebungen (29 und 29bis) versehen ist, die bei Druckausübung auf sie in Abhängigkeit von der Richtung und der Stärke die Beschleunigung des virtuellen Ablaufens der Schriftzeichen, den Stillstand des Ablaufens oder das Zurücklaufen bewirken.

9. Tragbare Lesevorrichtung für Blinde nach einem der vorhergehenden Ansprüche, **dadurch gekennzeichnet, daß** die Optik (4) in der Lage ist, die Schriftzeichen in einem Abstand zu lesen, wobei ein Autofokussystem oder die Hyperfokaleinstellung eines festen Objektivs verwendet wird.

10. Tragbare Lesevorrichtung für Blinde nach einem der vorhergehenden Ansprüche, **dadurch gekennzeichnet, daß** eine Abtastung jedes Moduls des Felds durch die Matrix von optischen Fühlern (5) diesem Modul, das einem oder mehreren Pixel zugeordnet ist, eine vordefinierte Tonfrequenz zuweist, die der dominanten Farbe dieses Moduls entspricht.

11. Tragbare Lesevorrichtung für Blinde nach einem der vorhergehenden Ansprüche, **dadurch gekennzeichnet, daß** der Sprung von einer Zeile zu einer anderen und von einem Bild zu einem anderen bei der Abtastung durch die Fühler (5) durch ein spezifisches Tonsignal oder eine sehr kurze Stille von einigen Millisekunden im Fall des Übergangs von einer Zeile zur anderen und von einigen zehntel Millisekunden im Fall des Übergangs von einem Bild zu einem anderen markiert ist.

12. Tragbare Lesevorrichtung für Blinde nach einem der vorhergehenden Ansprüche, **dadurch gekennzeichnet, daß** die optischen Fühler (5) die Erfassung von Infrarotlicht gestatten und dieses durch eine spezifische Tonfrequenz wiedergeben, die dem Blinden die Anwesenheit von Menschen oder Tieren anzeigt.

13. Tragbare Lesevorrichtung für Blinde nach einem der vorhergehenden Ansprüche, **dadurch gekennzeichnet, daß** die Leistung des akustischen Signals in Korrelation mit der jedem Modul (xₙyₘ) zugeordneten dominanten Farbe bei Annäherung an die Mittelachsen (33 und 34) gleichmäßig zunimmt und bei Entfernung von diesen gleichmäßig abnimmt.

14. Tragbare Lesevorrichtung für Blinde nach einem der vorhergehenden Ansprüche, **dadurch gekennzeichnet, daß** eine Matrix von optischen Fühlern auf Höhe jedes Ohrs angeordnet ist, wobei die Töne, die sich aus der Analyse der Signale durch die Matrix der am rechten Ohr angeordneten optischen Fühler (5) ergeben, zum rechten Ohr geleitet werden, und die Töne, die sich aus der Analyse der Signale durch die Matrix der am linken Ohr angeordneten optischen Fühler (5) ergeben, zum linken Ohr geleitet werden, was dem Blinden eine stereophone Wahrnehmung vermittelt.

15. Tragbare Lesevorrichtung für Blinde nach einem der vorhergehenden Ansprüche, **dadurch gekennzeichnet, daß** ein Bund die Versorgung der Elektronikeinheit (3) durch aufladbare Akkumulatoren sowie eine spezielle Schaltung umfaßt, die das Laden der Akkumulatoren durch einfache Einführung von Schließstiften (12) in eine Stromanschlußdose des Netzes gestattet.
